# EUROPEAN PATENT APPLICATION

(11) **EP 2 135 938 A1**
(43) Date of publication of application: **23.12.2009**
(21) Application number: 08425437.4
(22) Date of filing: 20.06.2008
(51) Int. Cl.: C12M 1/107, C12P 5/02

(54) **Anaerobic fermentation process and plant**

(71) Applicant: Trabattoni, Sergio, 26012 Castelleone (CR) (IT)
(72) Inventor: Trabattoni, Sergio, 26012 Castelleone (CR) (IT)
(74) Representative: Zardi, Marco

(57) **Abstract**

A process and related plant for the fermentation of an organic mass which provides for: a hydrolysis and acidogenic fermentation reactor (11) (which operates in aerobic or in anaerobic conditions); an acetogenic reactor (12); a methanogenic fermentation reactor (13); a recirculation (22) of at least a part of the residual biomass from the outlet of the methanogenic reactor to the acetogenic reactor or to the hydrolysis-acetogenesis, if conducted anaerobically, wherein the reactors are separated in order to provide optimal operating conditions for the different processes.

## Description

### Field of application

The invention regards a process and plant for the anaerobic fermentation of a plant and/or animal organic mass, in particular for the fermentation of sewage-manure with production of biogas.

### Prior art

Anaerobic fermentation is a known and widely studied process. The three fundamental steps of the process are a hydrolysis of the organic composts, an acid-acetogen digestion and a methanogen digestion. Such process steps involve different bacteria families, which optionally comprise anaerobic bacteria, active in the hydrolysis and in the acidogen step, and acetogen bacteria and methanogen bacteria which must instead be anaerobic.

In the anaerobic fermentation plants of known type, the aforesaid three process steps occur in a single digester. This plant solution is simple but has the disadvantage of requiring compromised operating conditions, compatible with the different bacteria families which coexist and cooperate in the same environment. The chemical-physical parameter values, such as pH, temperature, concentrations, redox potential, material exchange between liquid phase and gas phase, etc..., as well as the values of biochemical process parameters (acetogenesis, methanogenesis) are thus a compromise that does not fully meet the needs of the single bacterial families, with the result of not fully exploiting, in the best possible manner, the energy potentialities of the starting material (organic matrices) subjected to fermentation.

The processes and plants with single digester, moreover, have several use limitations. For example, they cannot be used with organic matrices which, during the process, lead to the formation of harmful products for specific bacterial families, in particular for the methanogens. This is inevitable in the prior art, in which the different bacteria must in fact coexist in the same digester. A single digester plant, moreover, cannot use matrices with high concentrations of suspended inert bodies, whose presence negatively influences both the mixing of the liquor and the gas collection. A strong presence of inerts in the liquor leads to a high energy consumption and mixing difficulty.

The supply material can be present with different chemical-physical characteristics, such that the plant must have high flexibility, not very common for traditional plants. This represents a limit for the diffusion of the fermentation plants herein considered.

In current technology, despite the growing interest in the field the aforesaid drawbacks have not yet been resolved in a satisfying manner, in particular for the production of biogas for energy purposes, which which is equivalent to a renewable energy resource.

### Summary of the invention

The objective underlying the invention is that of overcoming the drawbacks and limits of the prior art described above. The invention in particular is aimed to improving the efficiency of the known fermentation processes, as well as making possible the use of organic matrices with characteristics that do not allow their use in the current plants.

The problem is solved with a process of anaerobic fermentation of an anaerobic material, comprising at least the steps of:
a) feeding organic material into a first hydrolysis and acidogenic fermentation environment, with obtainment of an acid product;
b) feeding said acid product into a second anaerobic acetogenic fermentation environment, with obtainment of a liquor;
c) feeding said liquor to a third methanogenic fermentation environment, with obtainment of a biogas flow and a residue (digested) biomass;
said environments being separated and providing specific operating conditions for the respective processes of hydrolysis and acidogenic fermentation, acetogenic fermentation and methanogenic fermentation.

By "operating conditions", it is intended both chemical-physical or biochemical variables such as concentration, residence time, temperature, pH, etc... and the specific bacteria type for the respective above-listed processes.

According to one aspect of the invention, the process provides for a recirculation of at least one part of the residue biomass to the acetogenic fermentation environment or to the hydrolysis-acidogenic environment (if this operates anaerobically) of at least one residue biomass part. Such biomass recirculation, preferably, is such to maintain a concentration of about 20 kg/m3 of the bacteria in the acetogenic step. Depending on the supply type, part of the biomass leaving the methanogenic fermentation environment can be recycled as is, in particular if C/N>30 (carbon/nitrogen); in other cases, the biomass is filtered by obtaining a concentrate that is recycled in the acetogenic step, and a filtrate that is further treated.

Further aspects of the process according to the invention, which can be implemented based on needs, are the following.

The organic supply material can be diluted before it is introduced into the hydrolysis and acidogenic fermentation environment. If said material is solid, it is minced and water or liquid excrements are added in order to obtain a suspension at the desired value of organic dry matter (ODM).

The acid product obtained via hydrolysis and acidogenic fermentation can be subjected to a step of separating and/or reducing the suspended bodies, if necessary or deemed opportune.

The acidogenic hydrolysis-fermentation product can moreover be subjected to a filtration, preferably with *per se* known ceramic membrane, in order to avoid supplying, during the methanogenic step, metabolites with toxic effects for the methanogenic bacteria. This further filtration is advantageous, in particular, when the organic supply material is rich with salts and ammonia and/or protein nitrogen, and therefore could alter the osmotic equilibrium to the extent that it affects the reproduction speed of the methanogenic bacteria, or is rich with nitrogen to the extent that it could be harmful for the methanogenic bacteria.

According to a further aspect of the invention, the residual biomass from the methanogenic fermentation step is divided into three flows, of which: a first flow is subjected to a microfiltration process with obtainment of a concentrate which is recirculated to the acetogenic (or hydrolytic-acidogenic) fermentation step and a filtrate; a second flow is supplied to a pressing step, with obtainment of a biomass with a high percentage of total solid matter (TSM), adapted for example for farming use; a third flow is recycled as is to the hydrolytic-acidogenic step in order to maintain a correct concentration of nitrogen in the containers when there is a supply shortage thereof.

According to a further aspect of the invention, the filtrate deriving from the microfiltration of the first flow is subjected to a reverse osmosis process, with obtainment of a concentrate containing nitrogen. Said concentrate can be subjected to a removal treatment or recovery treatment of the nitrogen for agricultural use, or for correcting the carbon/nitrogen (C/N) ratio or alkalinity.

The pressing step is preferably carried out in a filter-press, to which reference will be made below. The filtrate at the liquid state obtained in the filter-press is preferably further microfiltered, and is also subjected to the reverse osmosis process. The biomass separated from the microfiltration process is advantageously recycled to the same filter-press.

In particular, a further object of the invention are several methods for recovering the nitrogen contained in the concentrate deriving from the reverse osmosis.

Preferred methods for the removal of the nitrogen are: a) catalytic elimination of the ammonia nitrogen which is transformed into simple nitrogen, which can be discharged into the atmosphere, or b) transformation of the ammonia nitrogen into simple nitrogen by means of the known biochemical treatments of nitrification and denitrification or nitrosation and denitrosation (Anammox).

The recovery of the nitrogen, according to a particularly preferred embodiment, is carried out with a process that comprises the following steps:
- the concentrate, rich in ammonia nitrogen, is cold-saturated with a carbon dioxide (CO2) flow, in a suitable carbonation tower;
- the nitrogen is fixed in the form of ammonium bicarbonate, which remains dissolved in concentrated solution;
- said concentrated solution is absorbed by the solid biomass obtained via pressing.

The CO2 flow used for the saturation is preferably obtained by supplying at least a part of the biogas to the carbonation tower, or said CO₂ flow is separated from the motor exhaust fumes or boiler fed with the same biogas.

Preferably, straw or other absorbing organo-mineral substances are added to the solid obtained via pressing, in order to obtain a shovellable farming amendment (28-30% TSM) which is rich with fertilising substances (N,P,K). Said material is usually in the form of "pies".

Further variants of the invention are the following.

If the organic supply material is rich with insoluble substances (for example, lignin), a filtration is advantageously provided upstream of the hydrolysis step and acidogenic fermentation step.

In the case of organic material with low C/N ratio (typically less than 15) and high amount of nitrogen in ammonia form, a further filtration is advantageously provided for with a membrane filter, upstream of the hydrolysis and acidogenic fermentation, for washing away and separating the soluble ammonia nitrogen. Such expedient can be usefully employed, for example, for the treatment of poultry waste.

With organic material with high C/N ratio, for example greater than 30, a direct and at least partial recirculation is advantageously provided from the methanogenesis environment to the acetogenic environment or hydrolytic-acidogenic environment. Such recirculation compensates for the possible lack of nitrogen in acetogenesis due to the high C/N ratio.

The process can be used for producing biogas with energy use, with combustion of the biogas in a boiler or cogenerator. In this case, a further aspect of the invention provides for recovering at least part of the CO2 contained in the discharge gases of the boiler or cogenerator, in order to increase the presence of bicarbonate and control the pH in the acetogenesis and/or methanation steps. The separation of the CO2 from the combustion gases can be operated with known adsorbers.

The invention also has as object a fermentation plant adapted to operate according to the above-described process, in the different variants.

In particular, a fermentation plant is object of the invention which comprises at least one hydrolysis and acidogenic fermentation reactor; at least one anaerobic acetogenic fermentation reactor; at least one anaerobic methanogenic fermentation reactor; in which said reactors are separated in order to provide different fermentation operating conditions.

Preferably, the plant also comprises a recirculation line of at least part of the residual biomass, as is or filtered, from the methanogenic reactor to the acetogenic fermentation reactor.

Several reactors can be employed, in series or in parallel, for each process step. Preferably, the acidogenic step is conducted in small tanks sequentially used with full-empty logic, so to affect relatively small masses from time to time.

Further elements of the plant (membrane filters, reverse osmosis section, carbonation tower etc....) are provided in preferred embodiments, in accordance with the implementation of the process according to one of the above-illustrated variants.

The invention essentially has the following advantages. The sub-division of the process in stages allows obtaining a greater bacteria population; it permits the selection and enrichment of different bacteria in each stage; it renders the process more stable through the control of the acetogenic step, which is a critical passage for the entire process; it avoids the accumulation of toxic material (VFA); it increases the yield of supplied COD. In addition, the high density of the specific bacteria present in the methanogenic digester makes the system more resistant to the negative effect of inhibiting substances.

The separation of the hydrolytic-acidogenic step from the acetogenic step allows obtaining specific advantages. It is possible to adjust the operating conditions (pH, temperature, process duration, mixing speed...) to the characteristics of the organic matrix treated with greater ease, with respect to the prior art, since operations take place over small masses, while in the prior art the entire mass is in a single digester. The process is thus more flexible and adaptable with regard to the supply matrix. It is possible, if necessary, to insert small insufflations of air in order to accelerate the biomass formation.

The invention also permits to reduce the suspended matter in the hydrolysed-acidified product, by means of a suitable filtration downstream. Thus, one avoids forming floating islands of solid organic material in the acidogenic reactor, which are favoured by the generation of gas, until a crust is formed above the liquid.

The division into steps permits inserting suitable filtering systems between the reactors, with the advantage of making the plant suitable for treating a wide variety of supply material. For example, a filter can be advantageously inserted between the acidogenic step and the acetogenic step, in order to retain suspended or floating solid bodies which are present in a significant amount with a vegetal supply, for example straw.

The recirculation by the bacteria mass renders the plant capable of working with high volume and mass loads, reducing the hydraulic retention times and digester volumes.

The invention also permits accurately dosage over time of the supply of the organic load, preventing that the addition of organic material causes thermal, pH or concentration shocks. The present invention, with an original sequence of the process operations, structured on the three classical steps (hydrolytic-acidogenic, acetogenic and methanogenic), allows overcoming the limits presented by the single digester plants and also solves the problem of disposal of the digested material with particular attention to the recovery and/or disposal of the nitrogen and to the discharge of the waters with respect to environmental protection laws. Overall, there is an improved energy yield with respect to the prior art.

These and other advantages will be clearer with the aid of the following description of a preferred and non-limiting embodiment, and with reference to the attached drawings.

### Brief description of the drawings

Fig. 1 represents the diagram of a fermentation plant operating according to the process of the invention.
Fig. 2 and 3 represent variants of the diagram of Fig. 1, as a consequence of the characteristics of the supply material and in particular of the nitrogen content.

### Detailed description of preferred embodiments

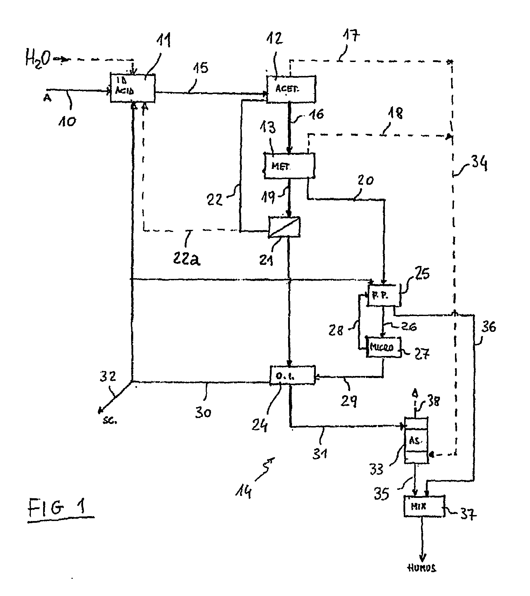

Fig. 1 shows a scheme of a treatment plant of a flow 10 of organic supply material A. Said material is composed, for example, of animal excrements, possibly subjected to pre-treatment such as mincing of solid parts, dilution with water if necessary, etc...., all this in order to attain a suitable value of organic dry matter (ODM). The scheme of Fig. 1 refers to the use of a material with average carbon/nitrogen value (CN ratio), approximately 15 - 30.

The plant essentially comprises a hydrolytic acidic reactor 11 which represents the hydrolysis and acidogenic fermentation environment; an acetogenic fermentation reactor 12 and a methanogenic fermentation reactor 13. Downstream of said reactor 13, there is a recovery and treatment section indicated with 14 in its entirety. The scheme of the figures is simplified and in particular does not comprise accessory equipment such as pumps, valves, etc.... which will be provided depending on the needs and known art in the field.

The reactor 11 generates an acid product which, by means of the flow line 15, is fed to the subsequent reactor 12. The mass 15 can be subjected to filtration, preferably with ceramic membranes, if the material A in the flow 10 is very rich both in salts and ammonia and/or protein nitrogen, as will be explained in detail below. Such filtration essentially consists of controlling, in the subsequent steps, the concentration of the nitrogen and salts, on which the osmotic and water equilibrium of the cells depend along with the speed of bacteria reproduction.

The product or liquor of the acetogenic fermentation (line 16) is fed to the methanogenic reactor 13 downstream. In the acetogenic fermenter, there is also a production of biogas, due to the presence of methanogenic bacteria, in particular hydrogenotrophic and acetoclastic bacteria. The biogas is drawn through the line 17.

The reactor 13 obtains a substantial production of biogas, collected by means of the line 18; the residual biomass is sent to the section 14 and partially recycled, through a first flow 19 and a second flow 20.

The first flow 19 is fed to a concentration filter 21, preferably of tubular fibre type, obtaining a concentrate 22 containing the bacteria mass, recycled at the acetogenic reactor 12 or at the hydrolytic-acidogenic reactor 11, and a filtrate 23 supplied to a reverse osmosis unit 24. Advantageously, the concentrate 22 contains 15-20% bacteria mass, and the flow rate is such to maintain about 20 kg/m³ of bacteria concentration in the acetogenic reactor 12.

The second flow 20 exiting from the reactor 13 is supplied to a filter-press 25, which obtains panels (pies) with 45-50% TSM usable for example as farming amendment. The liquid product 26 of the pressing is subjected to microfiltration in a suitable filter 27, with recirculation (line 28) of the solids to the same filter-press it is then fed to the reverse osmosis unit 24 through the line 29.

At output of the reverse osmosis, a permeate 30 and a concentrate 31 are obtained. The permeate 30 is almost exclusively represented by water, which is recycled for example to dilute the supply mass A, or sent to other uses or to a discharge 32; the concentrate 31 is rich in soluble salts and ammonia nitrogen, and for example is fed to a carbonation tower 33 where it is saturated with carbon dioxide by using the biogas produced, fed with the line 34 which collects the lines 17 and 18.

In the tower 33, a concentrated solution 35 is obtained of ammonium bicarbonate, which is preferably added to the panels or pies produced in the filter-press (line 36) in a mixer 37, obtaining an farming amendment that is particularly rich in fertilising substances (nitrogen, phosphorus, potassium).

This expedient has two important advantages: the ammonia is stabilised in the form of ammonium bicarbonate in concentrated solution. The use of biogas as fixer of the ammonia in the tower 33 avoids the hazards and costs connected with the use of other chemical reagents (for example sulphuric acid). The biogas is collected at the line 38 at the outlet of tower 33.

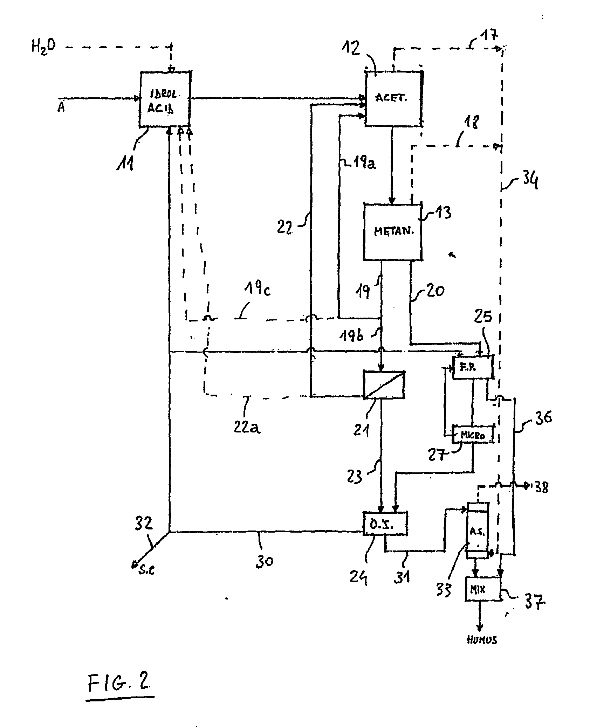

Fig. 2 represents a variant for material A with high nitrogen content, typically with C/N ratio greater than 30. In order to remedy the possible shortage of nitrogen in the acetogenic reactor 12 or in hydrolysis, a further recirculation of the biomass discharged from the methanogenic reactor 13 is provided. A part 19a of the flow 19, intended for the recirculation, is sent directly (as is) to the acetogenic reactor 12 or to the hydrolytic-acidogenic fermenter, while the remaining part 19b passes into the filter 21 and forms the recirculation flow. The other components of the plant, where indicated with the same reference numbers, are equivalent to those of Fig. 1.

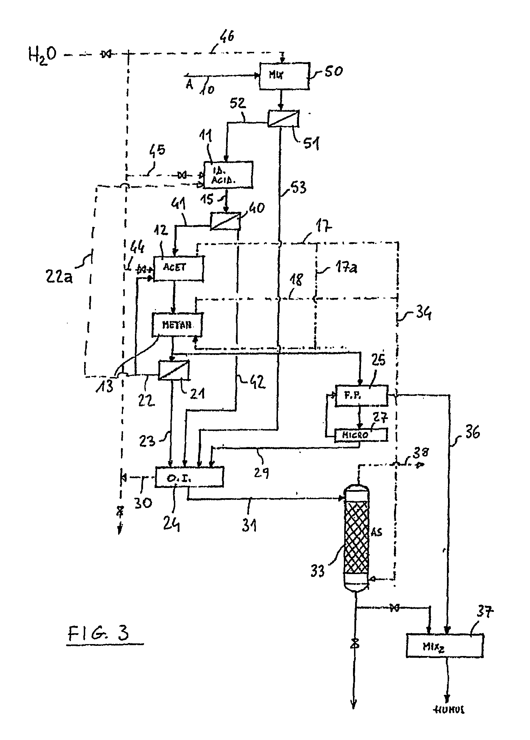

Fig. 3 represents a variant for the use with supply material A with very high nitrogen content (low C/N ratio). In order to avoid a high nitrogen content in methanogenic step, a further filter 40 is provided for between the acido-hydrolytic reactor 11 and the acetogenic reactor 12. Such filter obtains a concentrate 41, supplied to the acetogenic reactor 12, and a filtrate 42 sent to the reverse osmosis unit 24. In addition, a gas portion (line 17a) is preferably recirculated to the reactor 13.

The supply material A is mixed with water 46 in a quick mixer 50 upstream of the acido-hydrolytic reactor 11, with filtration in a filter 51. The filter 51 obtains a concentrate 52 fed to the reactor 11 and a filtrate 53 fed to the reverse osmosis 24. Also the intermediate water dilution lines 44 and 45 are shown, of the reactors 11 and 12, respectively.

If the acido-hydrolytic reactor 11 operates in anaerobic conditions, it can be alternatively provided a recirculation of biomass exiting from the reactor 13, along lines 22a and 19c, directed to said reactor 11.

The process is now described with greater technical detail, in reference to the various steps.

### Hydrolytic - acidogenic step

The organic material A, after possible pre-treatment in order to obtain the desired concentration of organic dry matter (ODM), is sent to tanks where, due to the presence of extracellular enzymes, inoculated and/or produced by hydrolysing self-forming and/or recycled bacteria, there is the quick transformation of the complex organic molecules of the substrate into monomers (fatty acids, glycerine, alcohols, amino acids, simple sugars); the latter are used by the bacteria to produce acetic acid, acids and alcohols with more than two carbon atoms, CO2, ammonia. The temperature normally ranges from 35 - 50°C.

In this step, characterised by a high production of acids and CO2, it is opportune that the pH lies in the range of 5.2-6.5. For this to occur, the buffer effect of the bicarbonates formed in the liquor or added with the supply material is of fundamental importance.

The hydrolytic - acidogenic step is preferably carried out in small tanks, used in the full-empty logic sequence, so to affect relatively small masses from time to time. It is thus easier to ensure a good mixing of the fed mass (fundamental for reducing the formation of VFA) and to set the operating conditions (concentrations, pH, temperature, duration of the process, mixing speed...) with the characteristics of the treated organic matrix.

The separation of this step from the acetogenic step permits the following advantages, among others:

If necessary, one can also use small insufflations of air in order to speed up the formation of biomass and/or favour the formation of bacteria attachment points with regard to hard-to-ferment organic molecules (for example, saturated animal fats). The operation is possible given that the hydrolytic-acidogenic bacteria are optionally anaerobic. In such case, however, the recirculation of the bacteria from the methanogenic step will have to be sent into the acetogenic step.

It is possible to interpose, between the acidogenic step and the acetogenic step, a filter for treating suspended or floating solid bodies which downstream, in the acetogenic reactor, could form a crust atop the liquid, especially in the case of vegetal supply.

The supply and temperature of the acetogenic bioreactor can be accurately programmed so that the fluid mechanics time therein, i.e. the residence time of the substrate (VFA), and the biochemical time, i.e. the time employed by the biophase to transform the substrate, which as is known is influenced by the temperature, at the very least coincide.

### Acetogenic step

The bacteria in question are: acetogenic bacteria, which transform the metabolites produced by the acidogenic bacteria into acetates, hydrogen and carbon dioxide; homoacetogenic bacteria, which produce the acetate ion, making the carbon dioxide and the hydrogen react; hydrogenotrophic methanogenic micro-organisms, which produce methane by using the carbon dioxide and the hydrogen dissolved in the liquor.

All of these operations occur inside the reactor 12, and in operating conditions or temperature, pH, mass load and volume load, hydraulic retention time, residence time of the bacteria, stirring speed, etc... such to be compatible with the bacteria characteristics. The partial recirculation 22 and 19a (if provided) of the biomass exiting from the methanogenic digesters has the object of maintaining the concentration of the bacteria high and allowing them a suitable residence time (bacteria age).

The biomass recirculation operated by means of line 34 essentially permits the following advantages: a) reducing the volume of the bioreactor; b) operating with effectiveness in the transformation of the propionate and butyrate ions into acetate ions, reducing the risk that volatile fatty acids (VFA) pass into the methanogenic digesters in quantities such to be toxic for the micro-organisms that produce methane; c) extending the residence time of the bacteria in the digesters and thus reducing the formation of muds, with the double advantage of limiting the problem of their disposal and reducing the quantity of COD used by them for their self-reproduction, and thus subtracted from the methane production.

### Filtration downstream of the hydrolytic-acidogenic reactor

This operation, not always necessary, can nevertheless be indispensable when the organic matrix A fed in the plant is particularly rich with salts and ammonia and/or protein nitrogen. The following circumstances must in fact be considered.
a) The saline concentration has little effect on the efficiency of the bacteria growth, but plays a role on the osmotic equilibrium, and thus on the water balance between the cell and the outside environment. The saline concentration thus affects the reproduction speed of the bacterial mass, so that the "tonicity" of the solution is a parameter to keep under control. In addition, the filtration can reduce the presence of heavy metal ions (for example Cd) possibly contained in the supply in concentrations such to be harmful.
b) Following metabolic processes operated by acidogenic bacteria, the nitrogen of the matrix, apart from the anabolic part of the bacteria themselves, was entirely transformed into ammonia nitrogen. This nitrogen, if present as NH3 in concentrations of 1200-1300 ppm, is toxic-harmful in particular for the methanogenic micro-organisms. With the separate step process, object of the invention, the toxicity limit of free NH3 is considerably greater (up to 4000 ppm); nevertheless, it may be opportune or necessary to eliminate a part thereof with supplies that are very rich in nitrogen.

The use of ceramic membranes for said filtration is preferred. This allows separating the liquor coming from the bioreactor into two flows: a microfiltrate flow - without suspended bodies and substantially free of organic molecules, in which most of the nitrogen and dissolved salts are contained - to be sent to the reverse osmosis; a flow recirculated into the acetogenic reactor, which contains all of the solid material already suspended in the liquor (mass of the bacteria and insoluble substances), all the organic material (VFA, aromatic acid alcohols, branched chain organic molecules,...), and part of the solution in the desired volume for bringing the necessary nitrogen and salts into the subsequent acetogenic and methanogenic steps.

The choice of the ceramic membranes as filtration means allows controlling the concentration of the nitrogen and salts in the downstream digesters, without causing any thermal shock for the bacteria as occurs with other known methods (for example ammonia stripping). The osmotic work and water equilibrium of the cells depends on the salts, as does their generation speed. Consequently, the invention makes possible the production of biogas even from organic matrices with C/N ratios that are too low or with a high saline content, which in the prior art would be unusable as process supply unless appropriate corrections are made, which however involve difficulties and additional costs.

### Acetogenic digester

Some products of the fermentation such as the fatty acids with chain with two or more carbon atoms, alcohols with more than one carbon atom, products with branched chains and aromatic fatty acids cannot be used directly in methanogenesis.

In the acetogenesis process, these products are oxidised to acetates and hydrogen by the bacteria which are obligated hydrogen producers (OHPA), which operate in syntrophic relation with the hydrogenotrophic methanogenic bacteria. In consideration of the fact that the efficiency of the hydrogen use is greater the smaller the distance between the bacteria which produce it and those which use it, maximum efficiency is achieved when the OHPA and the hydrogenotrophic methanogenic bacteria are aggregated together.

The acetogenic bacteria do not only make use of the syntrophic action of the hydrogenotrophic methanogenic bacteria, but also benefit from the action of the acetoclastic methanogenic bacteria since these, by removing the acetate ions, favour the flow of the oxidation acetogenic reactions of the VFAs.

In order to favour syntrophism and thus the efficiency of the metabolism of the various bacterial families, it is advantageous to operate in acetogenic step with a pH range of 6.5 - 7.5, and to add granules of suitable inert material. Said inert material must be hydrophobic, it must have a surface area per volume unit and a specific weight approximately equal to that of the liquor in which it is introduced. Around the inert granules, there is the stratified formation of methanogenic bacterial film, then acetogenic material film and finally acidogenic material film. For the process to take place, it is necessary that the environment is in a nearly hydrodynamic stasis; preferably, the relative speed of the granules with respect to the surrounding liquid is <0.15 m/h.

If, for the characteristics of the supplied organic matrix, the introduction of filter 40 is not necessary (Fig. 3) for reducing the nitrogen or salts, the flow exiting from the hydrolytic-acidogenic step 11 is sent directly to the acetogenic digester 12.

If, instead, the filtration is necessary for controlling the nitrogen and/or salts, only the concentrate coming from the filtration (line 41) passes into the acetogenic digester, where it is diluted with recovery water (line 44) so to bring the bacterial concentration, the mass and volume load, the salinity, and the concentration to the desired values.

The operating conditions (pH, temperature, mixing speed, retention time...) must account for the fact that next to the acetogenic bacteria in this step, also the hydrogenic methanogenic bacteria must be present, and even if in a small amount, as well as the acetoclastic methanogenic bacteria, with which such acetogenic bacteria must operate in syntrophy, for at least two reasons:
1) disposing the hydrogen produced by the oxidation of the VFAs into acetic acid; otherwise, the anaerobic fermentation is blocked, since the contribution of the homoacetogenic bacteria in the use of the hydrogen is quite negligible with respect to that of the hydrogenotrophic methanogenic bacteria;
2) reducing the concentration of the VFAs in the liquor to be sent to the actual methanogenic step to a minimum.

### Methanogenic digesters

The liquor which from the acetogenic step passes into the methanogenic step contains all the acetate ions and all the hydrogen which the few methanogenic bacteria present in that step were unable to methanise.

In this step, there is the production of biogas particularly rich in methane. In order to favour the methanation, it is necessary to provide for a correct retention time and an age of the bacteria that allows their generation, and permits a high formation of bacteria granules. In order to have a good methane yield, it is fundamental that the bacteria granules were sufficiently wetted by the surrounding liquor, in order to draw the necessary food therefrom.

Since a lot of insoluble gas (methane) is formed which could pull the granules towards the surface of the liquid (so-called aerostatic balloon effect, typical of flotation), it is necessary that the gaseous generation occurs via microbubbles. It is also important that the mixing speed of the liquid is such to not block nor disturb the formation of new granules and at the same time maintain uniform the concentration of the metabolites in the entire liquid mass of the digester.

With respect to the acetogenic bioreactor, in methanogenesis the pH tends to increase, even if preferably remaining between 7.5 and 8.5 in stable process conditions. In order to increase the kinetics of the biochemical reactions, if there isn't the risk of an excessive concentration of free NH3, the temperature can be slightly increased; the temperature increase, moreover, lowers the surface tension of the liquid and improves the wettability of the bacteria granules.

In order to maintain the NH3 concentration under control, when matrices rich in nitrogen are treated the invention provides for sending in the digester a flow of biogas such that the CO2 contained therein reacts with the ammonium ion to form the buffer NH4HCO3 Considering the fact that the methanogenic micro-organisms have doubling times considerably greater than those of the acidogenic and acetogenic bacteria, the water retention time and the residence time of the bacteria in this step are longer than those of the preceding steps.

### Filtration with partial recirculation of the bacterial mass

The filter 21, which treats the flow portion 19 exiting from the methanogenic digesters, preferably makes a microfiltration with tubular fibres, in order to obtain the concentrate 22 containing the suspended material (produced bacterial mass and inert material), to be recirculated, and the filtrate 23 to be sent to the reverse osmosis step.

In one variant, it is possible to use a normal filter, rather than a tubular filter, or a centrifuge; in such case, the filtrate 23 cannot go directly into reverse osmosis but, together with the unfiltered fraction 20, it must be sent to the filter-press 25.

### Press-filtering

The press-filtering aims to make the colloidal suspension which exits from the methanogenic digesters filterable. With the pressure-filtering, one obtains panels (pies) with 45-50% TSM, containing the bacterial biomass formed in the process and the inert solids.

In said panels or pies, nearly all of the phosphorus is retained, in precipitate form, which entered with the supply A, along with small amounts of potassium and nitrogen dissolved in the liquor with which the panels themselves were soaked. These pies are optimal amendments, which due to the strong water retention capacity (up to three times their own weight) they improve the capacity to retain water and that, due to the good chelating and ion exchange capacity, reduce the danger of percolation of the heavy metals present into the surrounding ground water table.

The liquid 26 exiting from the filter-press (filtrate), which contains the soluble salts contained in the supply and still has about 0.3 - 0.5 % suspended solid bodies, is sent to the microfiltration (filter 27) and subsequently subjected to reverse osmosis (line 29).

### Reverse osmosis

The following flows are subjected to reverse osmosis:
a) the liquid flow 42, rich with nitrogen and dissolved salts, coming from the filter-concentrator 40 possibly placed downstream of the hydrolytic acidogenic reactor, and possibly the flow 53 separated in the further filter 51 (Fig. 3);
b) the flow 23 coming from the tubular membrane filter-concentrator 21, placed downstream of the methanogenic digesters;
c) the filtrate 29 coming from the filter-press 25, previously microfiltered in the filter 27.

The outflow from the osmosis is represented by the permeate 30 and by the concentrate 31. Practically only water passes in the permeate 30, which is all or partly recycled, or sent to other industrial uses or discharged into the surface waterways; in the concentrate 31, the soluble salts and the ammonia nitrogen remain. Preferred treatment methods of the concentrate 31 are described below.

### Treatment of the concentrate from the reverse osmosis

The treatment can be aimed to eliminate the nitrogen, or recovering the nitrogen both for agronomic purposes and to be returned to the plant for improving a process parameter (C/N ratio, alkalinity).

In order to eliminate the nitrogen one of the following processes is preferably used: catalytic elimination of the ammonia nitrogen, which is transformed into simply nitrogen which can be discharged into the atmosphere, or transformation of the ammonia nitrogen into simple nitrogen by means of *per se* known biochemical treatments of nitrification-denitrification or nitrosation-denitrosation (Anammox).

For the recovery of the nitrogen, the invention preferably provides for one of the processes described below.

In a first embodiment, the concentrate 31 is cold-saturated, with the carbon dioxide contained in the biogas 34, in the carbonation tower 33. The nitrogen is thus fixed in the form of ammonium bicarbonate, which remains dissolved in the concentrated solution 35 recovered in the tower 33. This solution is then absorbed by the pies obtained from the filter-press, by means of the mixer 37. If necessary, straw or other absorbing organo-mineral substances are added.

According to one variant, CO2 is used in the tower 33, separated from the combustion gases of a boiler or motor fed with the produced biogas.

In a second embodiment, the concentrate 31 is in part fed to a nitrification tank, where the ammonia is oxidised to nitric acid. This acidic solution is joined with the remaining part, rich in ammonia, and there is the formation of an ammonium nitrate solution, advantageously made to be absorbed by the pies produced in the filter-press or used as is.

In a further embodiment, said concentrate 31 is treated with sulphuric acid, obtaining a solution of salts and ammonium sulphate; said solution is then fed to the reverse osmosis unit, obtaining water and a concentrated solution of salts and ammonium sulphate.

The nitrogen recovery, by means of the above-described processes, can be set to correct the C/N ratio of the supply and/or alkalinity of the liquor, fundamental parameters for a good operation of the process, if organic matrices having a shortage of nitrogen are used.

The process and plant as described attain the objects and solve the problems of the prior art mentioned above. A man skilled in the art, in order to satisfy specific and contingent needs, can make further modifications and variants which are considered to be within the protective scope of the present invention as defined by the following claims.

## Claims

1. Anaerobic fermentation process of an organic supply material, **characterised in that** it comprises at least the steps of:
- feeding the organic material into a first hydrolysis and acidogenic fermentation environment (11), with obtainment of an acid product;
- feeding said acid product into a second anaerobic acetogenic fermentation environment (12), with obtainment of a liquor (16);
- feeding said liquor (16) to a third methanogenic fermentation environment (13), with obtainment of a biogas flow (33) and a residual biomass;
and **in that** said hydrolysis and acidogenic fermentation, acetogenic fermentation and methanogenic fermentation are separated and provide specific operating conditions for the respective processes.

2. Process according to claim 1, comprising a step of recirculating at least a part (19a, 22) of said residual biomass, produced in the methanogenic fermentation, to said acetogenic fermentation environment (12) or to the first hydrolysis and acidogenic fermentation environment (11).

3. Process according to claim 2, wherein the residual biomass coming from the methanogenic fermentation is divided into:
- a first flow (19) subjected to a microfiltration process, with obtainment of a concentrate (22) which is recirculated to said acetogenic fermentation environment (12) or to the first hydrolysis and acidogenic fermentation environment (11);
- a second flow (20) subjected to a pressing step, with obtainment of biomass with high dry matter percentage.

4. Process according to claim 3, wherein the filtrate (23) obtained from said microfiltration process is subjected to a reverse osmosis step, with obtainment of a concentrate (31) containing nitrogen.

5. Process according to claim 4, wherein a liquid filtrate obtained in said pressing step is further microfiltrated and subjected to said reverse osmosis process.

6. Process according to claim 4 or 5, wherein said concentrate (31) containing nitrogen, obtained via reverse osmosis, is subjected to a treatment for eliminating or recovering the nitrogen contained therein.

7. Process according to claim 6, wherein said concentrate (31) containing nitrogen is subjected to a treatment for recovering the nitrogen by means of cold saturation with carbon dioxide in a carbonation tower (33), with obtainment of a solution containing nitrogen fixed in stable ammonium bicarbonate form.

8. Process according to claim 7, wherein said cold saturation in the carbonation tower (33) is operated with at least a part of the biogas containing carbon dioxide obtained in the methanation and/or acetogenic fermentation environment.

9. Process according to claim 7, wherein a biogas flow produced in the methanation process is used for combustion in a boiler or cogenerator, and at least a part of CO2 contained in the exhaust gases of said boiler or cogenerator is separated for use in said carbonation tower (33) for the nitrogen recovery.

10. Process according to one of the claims 6 - 9, wherein a concentrated solution (35) containing ammonium bicarbonate, obtained from the recovery of the nitrogen contained in said concentrate (31), is used as additive for the solid biomass obtained via pressing.

11. Process according to any one of the preceding claims, further comprising a step of filtering the product (15) obtained in the hydrolytic-acidogenic environment (11), preferably with membrane filter, upstream of the subsequent acetogenic fermentation environment (12), said filtration step is adapted to reducing the concentration of salts and/or ammonia nitrogen entering into said environment (12).

12. Fermentation plant of an organic supply material (A), adapted to operate a process according to at least one of the preceding claims.

13. Plant according to claim 12, comprising:
- at least one hydrolysis and acidogenic fermentation reactor (11);
- at least one anaerobic acetogenic fermentation reactor (12);
- at least one anaerobic methanogenic fermentation reactor (13);
wherein said reactors (11, 12, 13) are separated in order to provide different fermentation operating conditions.

14. Plant according to claim 13, comprising a line (22, 19a) for recirculating at least a part of the residual biomass as is or filtered, from the methanogenic reactor (13) to the first acetogenic fermentation reactor (12), or to the hydrolysis-acidogenic step (11), the latter operating in anaerobic conditions.
